# EUROPÄISCHE PATENTSCHRIFT

(11) **EP 2 214 112 B1**
(45) Veröffentlichungstag und Bekanntmachung des Hinweises auf die Patenterteilung: **23.01.2013**
(21) Anmeldenummer: 09151686.4
(22) Anmeldetag: 30.01.2009
(51) Int. Cl.: G06F 19/00

(54) **Verfahren zum Festlegen einer Konstellation von Messpunkten auf einer anatomischen Struktur**
Method for attaching a constellation of measurement points to an anatomical structure
Procédé de fixation d'une constellation de points de mesure sur une structure anatomique

(43) Veröffentlichungstag der Anmeldung: 04.08.2010
(73) Patentinhaber: BrainLAB AG, 85622 Feldkirchen (DE)
(72) Erfinder: Tuma, Gregor, 81677, München (DE); Grünschläger, Frank, 80639 München (DE); Adamski, Martin, 81667, München (DE); Oschinski, Andreas, 80639 München (DE)
(74) Vertreter: Schwabe - Sandmair - Marx

(56) Entgegenhaltungen:
- US-A- 5 377 011
- US-A1- 2007 249 967
- YU ZHANG ET AL: "From Range Data to Animated Anatomy-Based Faces: A Model Adaptation Method" 3-D DIGITAL IMAGING AND MODELING, 2005. 3DIM 2005. FIFTH INTERNATIONAL CONFERENCE ON OTTAWA, ON, CANADA 13-16 JUNE 2005, PISCATAWAY, NJ, USA,IEEE, 13. Juni 2005 (2005-06-13), Seiten 343-350, XP010811015 ISBN: 978-0-7695-2327-9 Gefunden im Internet: URL:http://ieeexplore.ieee.org/stamp/stamp .jsp?arnumber=1443264&isnumber=31039>
- F.I. PARKE AND K. WATERS: "Computer Facial Animation" 1996, WELLESLEY , MASSACHUSETTS , XP002527620 * Seite 83 - Seite 84 *

## Beschreibung

Die vorliegende Erfindung betrifft ein Verfahren und eine Vorrichtung zum Festlegen einer Konstellation von Messpunkten auf einer anatomischen Struktur eines Patienten.

Patent Nr. US 5 377 011 offenbart unterschiedliche Abtastdichten in verschiedenen Regionen einer abzutastenden Struktur und ist somit als nächstliegender Stand der Technik anzusehen. Allerdings liegt hier kein Modell des abzutastenden Objekts vor.

Dokument "From Range Data to Animated Anatomy-Based Faces: A Model Adaptation Method", Yu Zhang et al., Proc. 3DIM'05, offenbart zwar ein generisches Modell der abzutastenden Struktur, unterteilt diese jedoch nicht in verschiedene Bereiche mit unterschiedlicher Komplexität und führt darüber hinaus eine äquidistante Abtastung durch.

In der medizinischen Anwendung ist es bekannt, ein generisches Modell einer anatomischen Modellstruktur, beispielsweise eines Knochens, auf die anatomische Struktur zu morphen. Eine anatomische Struktur ist eine Struktur eines Körpers, beispielsweise ein Knochen oder ein Organ eines Patienten. Das generische Modell der anatomischen Struktur dient der (approximativen) Beschreibung bzw. Wiedergabe der anatomischen Struktur.

Als Eingangswerte für den eigentlichen Morphing-Vorgang werden Messpunkte auf der Oberfläche der anatomischen Struktur mittels eines Abtastverfahrens abgetastet, beispielsweise unter Verwendung eines Pointers. Dadurch wurden Messpunktdaten erzeugt, die insbesondere die Position der Messpunkte bevorzugt in drei Dimensionen repräsentieren. Anschließend wird das generische Modell so verändert, dass es den abgetasteten Messpunkten entspricht. Bei dem Modell handelt es sich üblicherweise um ein Gittemetzmodell mit einer Vielzahl von Gitterpunkten, das auch als Point Distribution Model (PDM) bezeichnet wird.

Das Modell wird insbesondere von Modelldaten repräsentiert, wobei die Modelldaten bevorzugt die zwei- oder dreidimensionalen Positionen der Gitterpunkte umfassen. Das Modell ist beispielsweise ein statistisches Modell, das aus der Vermessung einer Vielzahl gleicher Strukturen gewonnen wird. Während des Morphens wird die relative Lage der Gitterpunkte zueinander derart angepasst, dass das Modell, insbesondere in Form der Modelldaten, bestmöglich mit den Messpunkten übereinstimmt, also die anatomische Struktur nachbildet. Dieser Vorgang, der auch als Matching bezeichnet wird, ist im Stand der Technik bekannt.

Je nach Art der anatomischen Struktur ist es notwendig, bis zu mehrere hundert Messpunkte abzutasten. Daraus ergibt sich ein beträchtlicher Zeitaufwand zur Abtastung der anatomischen Struktur. Es ist die Aufgabe der vorliegenden Erfindung, die für das Abtasten notwendige Zeit zu verringern.

Diese Aufgabe wird gelöst durch das Verfahren zum Festlegen von Konstellationsdaten gemäß Patentanspruch 1, das Softwareprogramm nach Patentanspruch 12, ein computerlesbares Medium nach Patentanspruch 13 sowie eine Vorrichtung zum Festlegen von Konstellationsdaten gemäß Patentanspruch 14. Die Erfindung betrifft weiterhin ein Verfahren zum Morphen eines generischen Modells auf eine anatomische Struktur eines Patienten gemäß Patentanspruch 10. Vorteilhafte Ausgestaltungsformen sind den abhängigen Ansprüchen zu entnehmen.

Erfindungsgemäß werden die Konstellationsdaten, die die Konstellation der Messpunkte repräsentieren, für verschiedene Bereiche der anatomischen Struktur individuell festgelegt. Dadurch ergibt sich für jeden Bereich die ideale Konstellation der Messpunkte. Dabei umfasst der Begriff Konstellation insbesondere die Anzahl und/oder den Abstand der Messpunkte und/oder die Anzahl der Messpunkte pro Flächeneinheit (z. B. cm²) der abgetasteten Fläche. Bevorzugt werden die festgelegten Konstellationsdaten als Konstellationssignale, die Konstellationsinforrnation umfassen, insbesondere von einem Computer ausgegeben. Die Konstellationsdaten sind die Gesamtheit der Konstellationsdaten aller (relevanten) Bereiche.

Die Messpunktdaten werden beispielsweise durch ein Abtastverfahren ermittelt und dann dem Computer über eine Schnittstelle zur Verarbeitung bereitgestellt. Die Konstellationsdaten werden dem Abtastverfahren bereitgestellt. Das Verfahren zur Festlegung der Konstellationsdaten und das Abtastverfahren sind üblicherweise separat durchführbare Verfahren.

Bei den bisherigen Abtastverfahren ist es üblich, die Messpunkte in einem statischen Punktraster abzutasten. Dabei werden unter Umständen unkritische Bereiche der anatomischen Struktur zu fein und kritische Bereiche der anatomischen Struktur zu grob abgetastet. Durch die bevorzugte Berücksichtigung von Konstellationsdaten wird es ermöglicht, verschiedene Bereiche der anatomischen Struktur jeweils mit der optimalen Punktanzahl und/oder der optimalen Punktdichte (also Anzahl pro Flächeneinheit) und/oder dem optimalen Punktabstand abzutasten und somit die Abtastung nicht benötigter Punkte zu verhindern.

Das erfindungsgemäße Verfahren ist insbesondere ein Datenverarbeitungsverfahren. Das Datenverarbeitungsverfahren wird bevorzugt durch technische Mittel, insbesondere einen Computer, durchgeführt. Der Computer umfasst insbesondere einen Prozessor und insbesondere einen Speicher, um insbesondere elektronisch die Daten zu verarbeiten. Insbesondere werden die beschriebenen Schritte des Berechnens bzw. Festlegens von einem Computer ausgeführt. Schritte des Definierens von z. B. Bereichen oder Werten sind insbesondere Schritte des Festlegens von Daten im Rahmen des technischen Datenverarbeitungsverfahrens, insbesondere im Rahmen eines Programms. Schritte des Ändems stellen insbesondere eine Änderung der Daten mittels des Computers dar.

Die Messpunkte werden vorzugsweise durch Messpunktdaten beschrieben. Die Konstellationsdaten beschreiben insbesondere die Anzahl der Messpunkte und/oder den Abstand der Messpunkte. Die Schritte des Festlegens und Ermittelns werden vorzugsweise von einem Computer insbesondere automatisch durchgeführt. Hierzu kann der Computer auch Berechnungen durchführen. Insbesondere erfolgt die Festlegung der Konstellationsdaten mit Hilfe eines Computers. Die genannten Messpunktdaten und Konstellationsdaten werden vorzugsweise bereitgestellt. Weiter werden vorzugsweise anatomische Strukturdaten bereitgestellt, die die anatomische Struktur beschreiben.

Werden hierin Daten, Regionen, Bereiche oder Bilder "bereitgestellt", so bedeutet dies, dass sie für die Nutzung durch das erfindungsgemäße Verfahren bereit sind. Diesen Zustand des "Bereitstehens" können die Daten, Regionen, Bereiche oder Bilder z.B. durch Erfassen der Daten, Regionen, Bereiche oder Bilder (z.B. durch Analysegeräte) oder durch Eingeben der Daten, Regionen, Bereiche oder Bilder (z.B. über Schnittstellen) erreichen. Die Daten können diesen Zustand auch dadurch haben, dass sie in einem Speicher (z.B. ROM, CD, Festplatte) abgespeichert und so für eine Verwendung im Rahmen des erfindungsgemäßen Verfahrens bereit stehen.

In einer beispielhaften Ausgestaltungsform der Erfindung bestimmt der Computer die Konstellationsdaten basierend auf der Komplexität des jeweiligen Bereichs und/oder mindestens eines benachbarten Bereichs. Je höher die Komplexität des Bereichs, desto größer wird die Anzahl der Messpunkte bzw. desto kleiner wird der Abstand der Messpunkte in diesem Bereich (automatisch) gewählt. Dabei werden die Komplexitäten anhand der anatomischen Struktur, des generischen Modells oder einer Kombination aus beidem bestimmt.

Dabei werden vorzugsweise Komplexitätsdaten verwendet, die die Komplexität von mindestens einem Bereich beschreiben. Die Komplexitätsdaten sind beispielsweise Daten, die Werte darstellen.

Für die Kategorisierung der Komplexität kann eine beliebige Skala verwendet werden, beispielsweise eine Skala mit Werten von 1 bis 10, von 1 bis 20, von 1 bis 50 oder von 1 bis 100, wobei der Wert 1 jeweils die kleinste Komplexität Skala bedeutet. Auch jede beliebige andere Quantifizierung der Komplexität ist möglich.

In einer beispielhaften Ausgestaltungsform der Erfindung werden Bereiche des generischen Modells der anatomischen Struktur bestimmt, die zu den Bereichen der anatomischen Struktur korrespondieren, und die Komplexität eines Bereichs der anatomischen Struktur wird aus der Komplexität des korrespondierenden Bereichs des generischen Modells ermittelt. Vorteilhaft werden die Komplexitäten der korrespondierenden Bereiche der anatomischen Struktur und des generischen Modells gleichgesetzt.

Somit werden die Informationen, auf denen die Festlegung der Konstellation der Messpunkte beruht, nicht oder nicht ausschließlich aus der anatomischen Struktur gewonnen, sondern (auch) aus dem generischen Modell. Der Zwischenschritt über die Komplexität des Bereichs der anatomischen Struktur kann weggelassen werden, sodass die Konstellationsdaten in einem Bereich der anatomischen Struktur direkt aus der Komplexität des korrespondierenden Bereichs des generischen Modells ermittelt werden.

Beispielsweise ist jedem Gitterpunkt des generischen Modells eine Komplexität zugewiesen und die Komplexität eines Bereichs des generischen Modells wird aus der Komplexität der Gitterpunkte in diesem Bereich ermittelt. Bevorzugt wird die größte Komplexität aller Gitterpunkte in dem jeweiligen Bereich des generischen Modells als die Komplexität des Bereichs der anatomischen Struktur bestimmt. Alternativ wird die Komplexität des Bereichs des generischen Modells als Mittelwert der Komplexitäten aller Gitterpunkte in dem Bereich ermittelt. Die Mittelwertbildung erfolgt ungewichtet oder gewichtet, beispielsweise wird höheren Komplexitäten bei der Mittelwertbildung ein höherer Gewichtungsfaktor zugewiesen als geringeren Komplexitäten.

In einem anderen Beispiel wird die Komplexität eines Bereichs des generischen Modells aus der Dichte der Gitterpunkte des generischen Modells in diesem Bereich bestimmt. Ist die Dichte der Gitterpunkte des generischen Modells hoch, so ist auch die Komplexität des Bereichs des generischen Modells und damit der anatomischen Struktur hoch und umgekehrt. Dies ist darin begründet, dass das Gitternetz des generischen Modells in Bereichen mit einem komplexen Oberflächenprofil der anatomischen Struktur üblicherweise dichter ist als in Bereichen mit geringerer Komplexität der Oberfläche. Als Maß für die Komplexität eines Bereichs des generischen Modells kann hier die Anzahl der Gitterpunkte in dem Bereich definiert werden, bevorzugt bezogen auf die Fläche des Bereichs.

Gemäß einer weiteren Ausgestaltungsform wird die Komplexität eines Bereichs des generischen Modells basierend auf der Varianz der zu dem generischen Modell gemittelten Abtastwerte der Strukturen in diesem Bereich ermittelt. Dies ist ein sinnvolles Kriterium, wenn das generische Modell ein statistisches Modell ist. Ein statistisches Modell wird durch eine Mittelwertbildung der Abtastwerte einer Vielzahl vermessener gleichartiger Strukturen ermittelt. Bei der Modellbildung wird also eine Vielzahl, beispielsweise in der Größenordnung von 50 bis 200, von Strukturen gleicher Art abgetastet und zu dem Modell gemittelt. Sind alle vermessenen Strukturen in einem Bereich identisch oder hochgradig ähnlich, so wird diesem Bereich des generischen Modells eine geringe Komplexität zugewiesen. Sind die vermessenen Strukturen in diesem Bereich stark unterschiedlich, so wird diesem Bereich eine hohe Komplexität zugewiesen. Beispielsweise sind in einer gespeicherten Nachschlagtabelle bestimmten Varianzwerten des generischen Modells bestimmte Werte der Komplexität zugeordnet und können vom Computer basierend auf Daten über das generische Modell mit Hilfe der Nachschlagtabelle automatisch bestimmt werden.
Optional wird das generische Modell anhand von Vorregistrierungsdaten, die Landmarken der anatomischen Struktur des Patienten repräsentieren, an die anatomische Struktur des Patienten angepasst und die Komplexität eines Bereichs der anatomischen Struktur aus der Komplexität des korrespondierenden Bereichs des derart angepassten generischen Modells ermittelt. Durch diese Verfahrensschritte erfolgt eine erste Grobanpassung des generischen Modells an die anatomische Struktur. Dies ist vorteilhaft, da eine anatomische Struktur je nach Patient eine unterschiedliche Größe und/oder Ausformung aufweisen kann. Außerdem wird das generische Modell optional dabei so orientiert, dass dessen virtuelle Lage mit der Lage der anatomischen Struktur übereinstimmt. Somit wird die Zuordnung eines abgetasteten Bereichs zu der korrespondierenden Stelle des generischen Modells vereinfacht.

Bei einer Landmarke handelt es sich um einen definierten, charakteristischen Punkt einer anatomischen Struktur, der bei der gleichartigen anatomischen Struktur mehrerer Patienten stets identisch oder mit hoher Ähnlichkeit wiederkehrt. Typische Landmarken sind beispielsweise die Epikondylen eines Oberschenkelknochens oder die Spitzen der Querfortsätze bzw. des Dornfortsatzes eines Wirbels.

In einer weiteren Ausgestaltungsform werden die Konstellationsdaten eines Bereichs aus einer Abbildung der anatomischen Struktur festgelegt. Bei der Abbildung handelt es sich beispielsweise um ein Röntgenbild, eine CT-Aufnahme, eine MRT-Aufnahme oder eine sonstige Abbildung, wie sie von einem bildgebenden Verfahren erzeugt wird. Durch eine Analyse der Abbildung wird ermittelt, welcher Bereich einer genaueren oder weniger genauen Abtastung bedarf und werden die Konstellationsdaten bzw. die Komplexität des Bereichs entsprechend festgelegt.

Die Analyse der Abbildung erfolgt entweder durch einen Bediener oder automatisch. Bei einer automatischen Auswertung wird die Abbildung beispielsweise mit einer korrespondierenden Abbildung, die sich aus dem generischen Modell ergibt, verglichen. Die Gewinnung einer zweidimensionalen Abbildung aus dreidimensionalen Daten, wie dem generischen Modell, ist als Digital Reconstructed Radiography (DRR) bekannt. Die Komplexität des Bereichs bzw. die Konstellationsdaten in diesem Bereich werden anhand der Abweichung der beiden Abbildungen in diesem Bereich festgelegt. Alternativ oder zusätzlich wird die Komplexität des Bereichs der anatomischen Struktur aus der Informationsdichte der Abbildung in diesem Bereich gewonnen. Die Informationsdichte ergibt sich beispielsweise aus der Energiedichte oder dem Frequenzspektrum, insbesondere dem Anteil hochfrequenter Bildanteile in der Abbildung bzw. dem Bereich der Abbildung.

Die Erfindung betrifft weiterhin ein Verfahren zum Morphen eines generischen Modells einer anatomischen Struktur auf eine anatomische Struktur eines Patienten anhand von Messpunktdaten, wobei die Konstellation der Messpunkte anhand eines Verfahrens wie vorstehend beschrieben bestimmt werden. Somit wird nicht nur die zum Abtasten notwendige Zeit verringert, sondern auch die Dauer des Morphing-Schrittes, da in diesem potentiell weniger Messpunkte berücksichtigt werden müssen. Die Messpunktdaten beschreiben die Messpunkte und damit die Oberfläche der anatomischen Struktur an den Messpunkten.

Optional wird das generische Modell beim Morphen an die Konstellationsdaten angepasst, zum Beispiel, indem die Anzahl und/oder Verteilung der Gitterpunkte des generischen Modells insgesamt oder in einem Bereich verändert wird. Dies bedeutet eine strukturelle Veränderung des generischen Modells, beispielsweise anhand der Messpunktdaten. Lässt sich das Modell in einem Bereich zum Beispiel nicht mit einer maximal zulässigen Abweichung auf die durch die Messpunktdaten beschriebene Lage der abgetasteten Messpunkte morphen, beispielsweise weil die anatomische Struktur in diesem Bereich eine komplexere Oberflächenbeschaffenheit aufweist als durch das Modell abbildbar ist, so wird das Modell um weitere Gitterpunkte ergänzt.

Die Erfindung betrifft weiterhin eine Vorrichtung zum Festlegen von Konstellationsdaten, die eine Konstellation von Messpunkten auf einer anatomischen Struktur repräsentieren, aufweisend einen Computer, der dazu ausgebildet ist, das Verfahren wie vorstehend beschrieben auszuführen. Der Computer legt also die Konstellationsdaten für jeden Bereich fest. Dazu ist das generische Modell in dem Computer oder in einer Speichereinheit, auf die der Computer zugreifen kann, hinterlegt.

Die Vorrichtung weist bevorzugt weiterhin eine Abtastvorrichtung zum Abtasten von Messpunkten auf der Oberfläche der anatomischen Struktur auf. Damit dient die Vorrichtung nicht nur zum Festlegen der Konstellationsdaten, sondern auch zur Durchführung des Abtastverfahrens. Bei der Abtastvorrichtung handelt es sich beispielsweise um einen Pointer. Der Pointer ist beispielsweise mit einer Markervorrichtung oder einem Referenzstern versehen, deren bzw. dessen Lage (also Position und/oder Ausrichtung) im Raum erfasst wird. Aus der Lage der Markervorrichtung bzw. des Referenzsterns und der relativen Lage der Spitze des Pointers zu der Markervorrichtung bzw. dem Referenzstern lässt sich die Position der Spitze des Pointers berechnen und als Messpunkt der Oberfläche der anatomischen Struktur verwenden.

Mit der Spitze des Pointers werden beispielsweise einzelne Punkte auf der Oberfläche der anatomischen Struktur abgetastet. Alternativ wird die Spitze über die Oberfläche bewegt und die Position der Spitze des Pointers in vorgegebenen zeitlichen oder räumlichen Abständen als Messpunkt festgehalten.

Aufgabe eines Markers ist, von einer Markererfassungseinrichtung (z.B. einer Kamera oder einem Ultraschallempfänger) erfasst zu werden, so dass seine Lage (d.h. Position und/oder Ausrichtung) im Raum ermittelt werden kann. Solche Marker können aktive Marker sein. Ein aktiver Marker emittiert beispielsweise elektromagnetische Strahlung bzw. Wellen, wobei diese Strahlung im infraroten, sichtbaren und/oder ultravioletten Spektralbereich liegen kann. Der Marker kann aber auch passiv sein, d.h. beispielsweise elektromagnetische Strahlung aus dem infraroten, sichtbaren und/oder ultravioletten Spektralbereich reflektieren. Dazu kann der Marker mit einer Oberfläche versehen sein, die entsprechende Reflektionseigenschaften besitzt. Es ist auch möglich, dass ein Marker elektromagnetische Strahlung bzw. Wellen reflektiert und/oder emittiert, die im Bereich der Radiofrequenzen oder bei Ultraschallwellenlängen liegt bzw. liegen. Ein Marker besitzt vorzugsweise sphärische Form bzw. eine kugelähnliche Gestalt und kann daher als Markerkugel bezeichnet werden, Marker können aber auch eine eckige, beispielsweise würfelige Gestalt aufweisen.

Ein Referenzstern bezeichnet eine Einrichtung, an der mehrere, vorteilhaft drei Marker angebracht sind. Die Marker sind dabei ortsfest und vorteilhaft lösbar an dem Referenzstern angebracht, so dass eine bekannte (vorteilhaft feste) relative Lage der Marker zueinander entsteht. Die relative Lage der Marker zueinander kann für jeden im Rahmen eines chirurgischen Navigationsverfahrens verwendeten Referenzstern individuell unterschiedlich sein, um anhand der relativen Lage der Marker zueinander eine Identifikation des dazugehörigen Referenzsterns durch ein chirurgisches Navigationssystem zu ermöglichen. Damit können dann auch die Gegenstände (z. B. Instrumente und/oder Körperteile) identifiziert bzw. voneinander unterschieden werden, an welche der Referenzstern angebracht ist. Der Referenzstern dient dazu, in einem chirurgischen Navigationsverfahren eine Mehrzahl von Markern an einem Gegenstand (beispielsweise einem Knochen oder einem medizinischen Instrument) anzubringen, um die Lage des Gegenstandes im Raum (d.h. seine Position und/oder Ausrichtung) erfassen zu können. Ein solcher Referenzstern umfasst insbesondere eine Anbringmöglichkeit an dem Gegenstand (beispielsweise eine Schelle und/oder ein Gewinde) und/oder ein Halteelement, das für einen Abstand der Marker von dem Gegenstand sorgt (um insbesondere die Sichtbarkeit der Marker für eine Markererfassungseinrichtung zu unterstützen) und/oder mit dem Halteelement mechanisch verbundene Markerhalter, an die die Marker angebracht werden können. Wo es aus dem Zusammenhang klar wird, kann der Begriff Referenzstern auch einen Referenzstern mit wenigstens einem daran angebrachten Marker bezeichnen. Ein solches System aus einem Referenzstern und wenigstens einem Marker kann auch Markerstern genannt werden.

Die Erfindung betrifft weiterhin ein Softwareprogramm, das, wenn es auf einem Computer ausgeführt wird oder auf einen Computer geladen wird, die Verfahrensschritte nach einem der vorstehend beschriebenen Verfahren ausführt, sowie ein computerlesbares Medium, auf dem ein solches Softwareprogramm gespeichert ist.

Im Rahmen der Erfindung können Computerprogrammelemente von Hardware und/oder Software (dies beinhaltet auch Firmware, im System abgespeicherte Software, Mikrocode usw.) verkörpert werden. Im Rahmen der Erfindung können Computerprogrammelemente die Form eines Computerprogrammprodukts annehmen, das durch ein auf einem Computer verwendbares oder computerlesbares Speichermedium verkörpert werden kann, das auf einem Computer verwendbare oder computerlesbare Programmanweisungen, "Code" oder ein "Computerprogramm" umfasst, die bzw. der bzw. das auf dem genannten Medium zur Verwendung auf oder in Verbindung mit dem System, das die Anweisung ausführt, verkörpert sind bzw. ist. Ein solches System kann ein Computer sein, ein Computer kann eine Datenverarbeitungsvorrichtung mit Mitteln zum Ausführen der Computerprogrammelemente und/oder des erfindungsgemäßen Programms sein. Im Rahmen dieser Erfindung kann ein auf einem Computer verwendbares oder computerlesbares Medium irgendein Medium sein, das das Programm zur Verwendung auf oder in Verbindung mit dem System, Apparat oder der Einrichtung, das bzw. der bzw. die die Anweisung ausführt, das Programm beinhalten, speichern, übermitteln, fortpflanzen oder transportieren kann. Das auf einem Computer verwendbare oder computerlesbare Medium kann z.B. ein elektronisches, magnetisches, optisches, elektromagnetisches, Infrarot- oder Halbleiter-System, ein solcher Apparat oder eine solche Einrichtung oder ein Ausbreitungsmedium wie z.B. das Internet sein, ist aber nicht auf diese Aufzählung beschränkt. Das auf einem Computer verwendbare oder computerlesbare Medium könnte sogar z.B. Papier oder ein anderes geeignetes Medium sein, auf das das Programm gedruckt ist, da das Programm elektronisch erfasst werden könnte durch z.B. optisches Scannen des Papiers oder anderen geeigneten Mediums und dann kompiliert, interpretiert oder andernfalls auf geeignete Weise prozessiert werden könnte. Das Computerprogrammprodukt und jegliche Software und/oder Hardware, das bzw. die hier beschrieben werden, bilden in den beispielhaften Ausführungsformen die verschiedenen Mittel zum Ausführen der Funktionen der Erfindung. Insbesondere kann der Computer bzw. die Datenverarbeitungsvorrichtung eine Hinweisinformationsvorrichtung darstellen, die Mittel zum Ausgeben einer Hinweisinformation beinhaltet. Die Hinweisinformation kann visuell durch ein visuelles Anzeigemittel (z. B. einen Monitor und/oder eine Lampe) und/oder akustisch durch ein akustisches Anzeigemittel (z. B. einen Lautsprecher und/oder eine digitale Sprachausgabeeinrichtung) und/oder taktil durch ein taktiles Anzeigemittel (z. B. ein in ein Instrument eingebautes vibrierendes Element bzw. Vibrationselement) beispielsweise an einen Benutzer ausgegeben werden.

Es ist im Rahmen der Erfindung möglich, einzelne oder mehrere Merkmale der vorstehend beschriebenen Ausgestaltungsformen, Alternativen und Beispiele miteinander zu kombinieren oder nicht zwingend notwendige Merkmale aus Merkmalskombinationen wegzulassen.

Die vorliegende Erfindung soll anhand eines Ausführungsbeispiels näher erläutert werden. Dabei zeigt
- Figur 1: ein generisches Modell mit drei markierten Bereichen,
- Figur 2: das generische Modell und vier Landmarken,
- Figur 3: das auf die Landmarken skalierte Modell,
- Figur 4: die Konstellationen der Messpunkte in den drei Bereichen und
- Figur 5: eine erfindungsgemäße Vorrichtung.

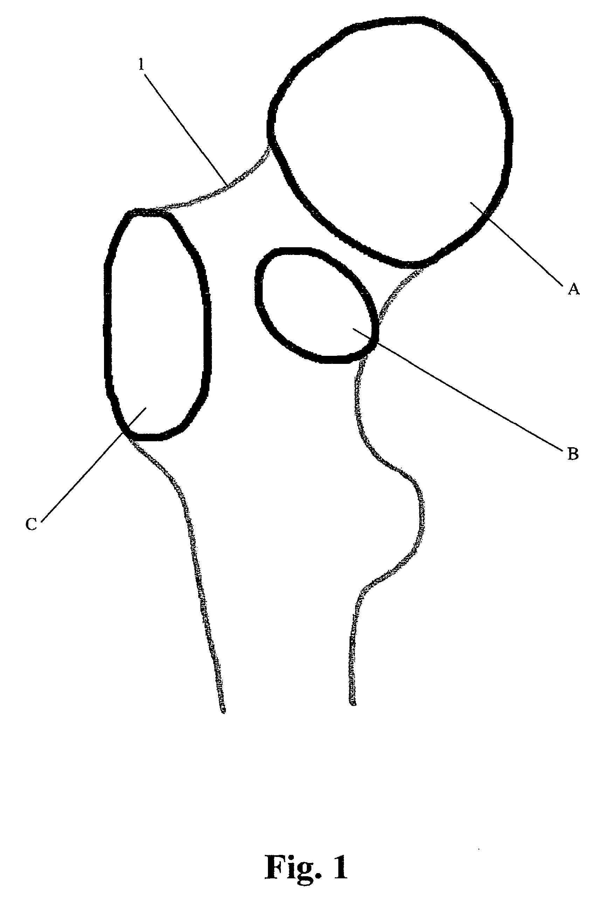

Die Figur 1 zeigt die Kontur einer zweidimensionalen Projektion des statistischen generischen Modells 1 eines Knochens. Das generische Modell 1 besteht aus einer Vielzahl von nicht dargestellten Gitterpunkten, wobei das Netz aus Gitterpunkten in seinem Ausgangszustand die durchschnittliche Form einer anatomischen Struktur, im vorliegenden Fall eines Oberschenkelknochens, nachbildet. Mit Hilfe eines Morphing-Verfahrens wird das generische Modell 1 verändert, um eine anatomische Struktur eines konkreten Patienten möglichst genau wiederzugeben. Das Morphing-Verfahren verwendet Messpunktdaten, die abgetastete Messpunkte auf der Oberfläche der anatomischen Struktur repräsentieren. Mittels des erfindungsgemäßen Verfahrens wird dabei zunächst für jeden abzutastenden Bereich die Konstellation der Messpunkte festgelegt.

Dazu werden zunächst beispielhaft drei Bereiche A, B und C des generischen Modells 1 ausgewählt, die für eine medizinische Anwendung von besonderem Interesse sind, beispielsweise um dort eine Veränderung des Knochens vorzunehmen oder ein Implantat zu befestigen. Da die Oberflächenbeschaffenheit des Knochens in den Bereichen, die zu den drei Bereichen A, B und C korrespondieren, unterschiedlich komplex sein kann, werden die zugehörigen Bereiche des Knochens mit einer unterschiedlichen Genauigkeit abgetastet. Für jeden der Bereiche des Knochens werden die Konstellationsdaten, die die Konstellation der Messpunkte repräsentieren, individuell festgelegt, das bedeutet in Abhängigkeit von der benötigten Abtastgenauigkeit.

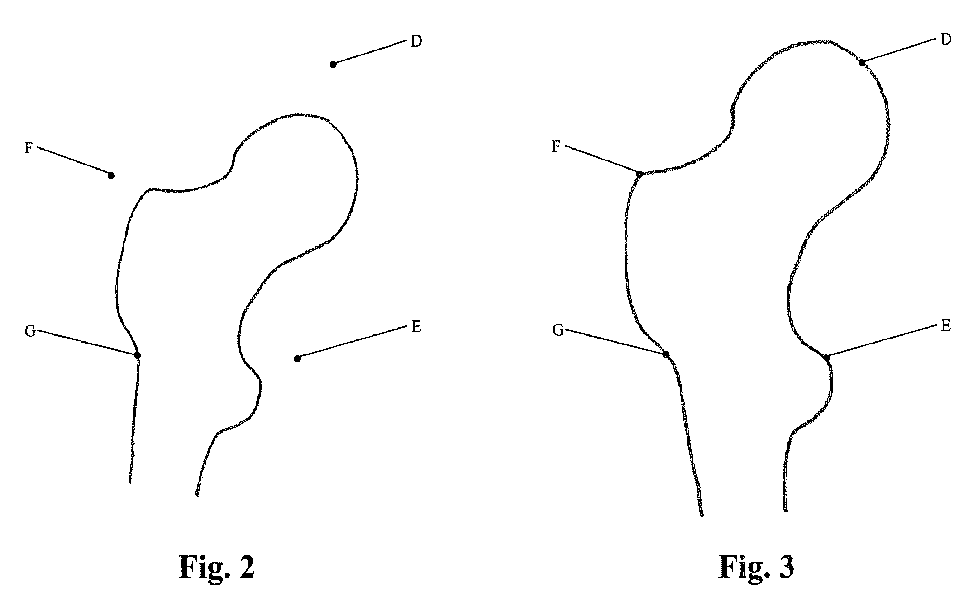

Dazu wird zunächst eine Vorregistrierung durchgeführt, bei der zum Beispiel vier Landmarken D, E, F und G des Knochens abgetastet werden, um Vorregistrierdaten zu erzeugen, die dann vom erfindungsgemäßen Verfahren zur Festlegung der Konstellationsdaten genutzt werden. Wie aus Figur 2 ersichtlich ist, stimmen diese abgetasteten Landmarken nicht zwangsläufig mit den korrespondierenden Landmarken des Modells 1 überein. Daher erfolgt gemäß dem Verfahren eine Skalierung und Ausrichtung des Modells 1 derart, dass die abgetasteten Landmarken D bis G mit den korrespondierenden Landmarken des Modells 1 bestmöglich übereinstimmen. Dieser Zustand ist in Figur 3 dargestellt. Bei diesem Schritt wird gleichzeitig eine räumliche Beziehung des Modells 1 zu den Landmarken D bis G des Knochens hergestellt. Dadurch wird später die Zuordnung eines abgetasteten Messpunkts des Knochens zu einem oder mehreren Gitterpunkten des generischen Modells 1 ermöglicht.

Bevor die Vorschläge für die Abtastung der drei Bereiche des Knochens ausgegeben werden, wird für jeden der Bereiche die Konstellation der zur Abtastung vorgeschlagenen Messpunkte in diesem Bereich individuell festgelegt. Die Konstellation der vorgeschlagenen Messpunkte umfasst den Abstand der Messpunkte und somit die Anzahl der Messpunkte innerhalb des jeweiligen Bereichs. Dazu wird jedem der drei Bereiche eine Komplexität zugewiesen. Die Komplexität jedes Bereichs wird aus dem Modell 1 abgeleitet. Jedem Gitterpunkt der Bereiche A, B und C des generischen Modells 1 ist eine Komplexität zugewiesen oder es wird die Komplexität aus der Dichte der Gitterpunkte des generischen Modells 1 in dem jeweiligen Bereich bestimmt. Die Komplexität eines Bereichs des Knochens entspricht der Komplexität des korrespondierenden Bereichs des generischen Modells 1. Alternativ wird die Komplexität eines Bereichs des Knochens aus Abbildungsdaten gewonnen, die eine Abbildung des Knochens, beispielsweise in Form eines Röntgenbildes oder eines MRT-Bildes, umfassen.

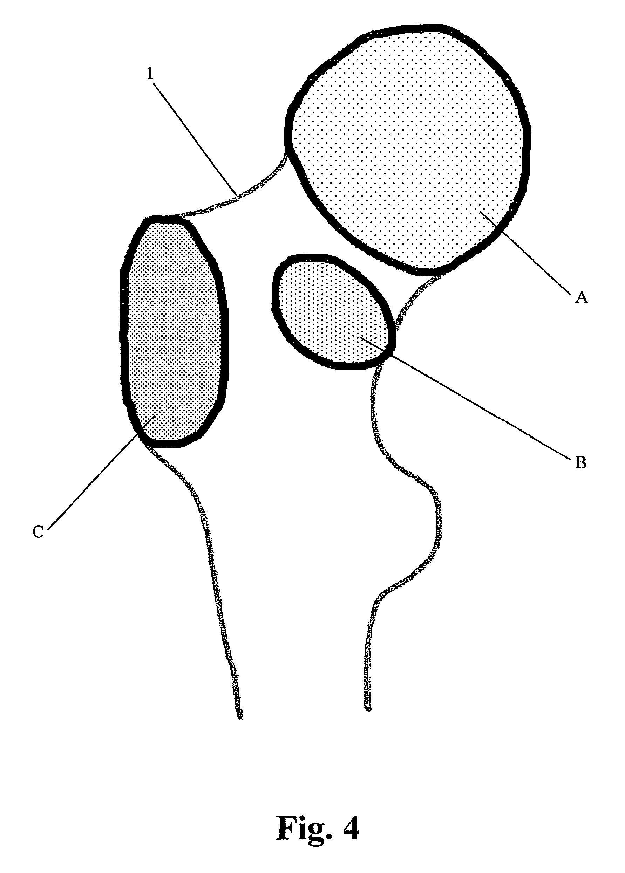

Im vorliegenden Beispiel weist der Bereich A eine geringe Komplexität auf, was zu einer geringen Dichte der Messpunkte in dem korrespondierenden Bereich des Knochens führt. Der Bereich B hat eine mittlere Komplexität und der Bereich C eine hohe Komplexität. Entsprechend ist die Dichte der Messpunkte in dem zum Bereich B korrespondierenden Bereich des Knochens mittel und in dem zum Bereich C korrespondierenden Bereich des Knochens hoch. Dies ist in der Figur 4 durch unterschiedlich dichte Füllungen der Bereiche A, B und C dargestellt.

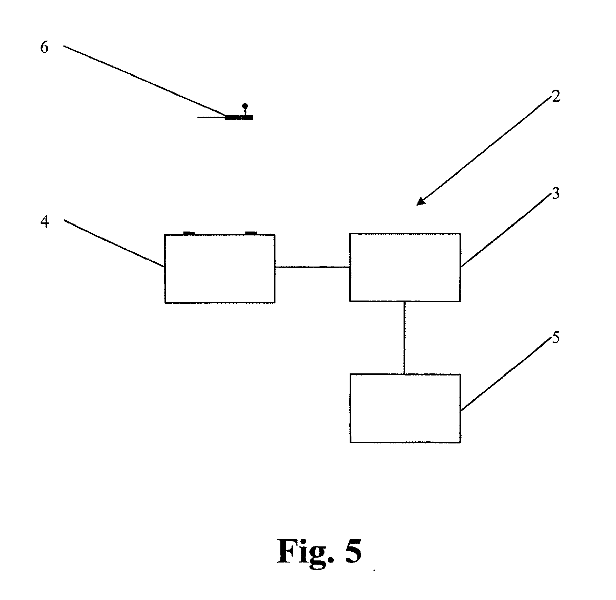

Die Figur 5 zeigt eine erfindungsgemäße Vorrichtung 2 zum Festlegen von Konstellationsdaten, die eine Konstellation von Messpunkten auf einer anatomischen Struktur repräsentieren. Die Vorrichtung weist einen Computer 3 auf, in dem das generische Modell 1 des Knochens hinterlegt ist. Der Computer 3 ist mit einem Bildschirm 4 und einer 3D-Kamera 5 verbunden. Die Kamera 5 dient der stereoskopischen Erfassung eines Pointers 6. Der Computer ermittelt die Lage, also die Position und/oder Ausrichtung, des Pointers 6 und dessen Spitze.

Mittels des Pointers 6 werden die Landmarken D, E, F und G des Knochens abgetastet und dem Computer 3 als Landmarkendaten bereitgestellt. Der Computer 3 berechnet dann auf Basis des vorstehend beschriebenen Verfahrens die Konstellationsdaten, also die Konstellation der Messpunkte in den Bereichen A, B und C.

Mittels eines Abtastvorgangs werden die drei Bereiche des Knochens beispielsweise mittels des Pointers 6 und gemäß der Konstellation der Messpunkte in dem jeweiligen Bereich abgetastet und Messpunktdaten erzeugt, die dann von einem nachgeordneten Verfahren genutzt werden, um das generische Modell 1 auf den Knochen zu morphen. Anhand der Vorregistrierung kann ein von den Messpunktdaten beschriebener Messpunkt einem der drei Bereiche A bis C zugeordnet werden. Bevorzugt wird dem Bediener, der die Abtastung vornimmt, der abzutastende Bereich des Knochens von dem Verfahren oder der Vorrichtung 2 kenntlich gemacht. Dazu wird beispielsweise das generische Modell 1 auf dem Bildschirm 4 dargestellt und der abzutastende Bereich optisch auf dem Bildschirm 4 hervorgehoben. Bevorzugt wird dem Bediener weiterhin angezeigt, wenn in einem Bereich ausreichend viele Messpunkte abgetastet wurden.

Auf Basis der Messpunktdaten, die die abgetasteten Messpunkte der Oberfläche des Knochens darstellen, wird die Lage der Gitterpunkte des generischen Modells 1 so variiert, dass das generische Modell 1 bestmöglich mit der Oberfläche des Knochens übereinstimmt. Verfahren für diesen Schritt, der als Morphing oder Matching bezeichnet wird, gehören zum Stand der Technik und sind dem Fachmann daher bekannt. Das Morphing wird beispielsweise ebenfalls vom Computer 3 durchgeführt.

## Patentansprüche

1. Verfahren zum Festlegen von Konstellationsdaten, die die Anzahl und/oder den Abstand von abzutastenden Messpunkten auf einer anatomischen Struktur eines Patienten repräsentieren, wobei die Konstellationsdaten für verschiedene Bereiche der anatomischen Struktur individuell festgelegt werden, wobei das Verfahren ein Datenverarbeitungsverfahren ist, das von einem Computer durchführt wird und die Festlegung der Konstellationsdaten für einen Bereich der anatomischen Struktur anhand der Komplexität des jeweiligen Bereichs und/oder mindestens eines benachbarten Bereichs erfolgt, wobei die Komplexität ein einem Bereich zugeordneter Wert ist und je höher die Komplexität eines Bereichs ist, desto größer wird die Anzahl der Messpunkte beziehungsweise desto kleiner wird der Abstand der Messpunkte in diesem Bereich gewählt, **dadurch gekennzeichnet, dass** Bereiche (A, B, C) eines generischen Gitternetzmodells (1) der anatomischen Struktur bestimmt werden, die zu den Bereichen der anatomischen Struktur des Patienten korrespondieren, einem Bereich des generischen Modells eine Komplexität zugewiesen ist, und die Komplexität eines Bereichs der anatomischen Struktur gleich der Komplexität des korrespondierenden Bereichs des generischen Modells (1) gesetzt wird.

2. Verfahren nach Anspruch 1, **dadurch gekennzeichnet, dass** das generische Modell (1) aus Gitterpunkten besteht, jedem Gitterpunkt eine Komplexität zugewiesen ist und die Komplexität eines Bereichs (A, B, C) des generischen Modells (1) aus der Komplexität der Gitterpunkte in diesem Bereich ermittelt wird.

3. Verfahren nach Anspruch 1, **dadurch gekennzeichnet, dass** das generische Modell (1) aus Gitterpunkten besteht und die Komplexität eines Bereichs (A, B, C) des generischen Modells (1) aus der Dichte der Gitterpunkte in diesem Bereich ermittelt wird.

4. Verfahren nach einem der Ansprüche 1 bis 3, **dadurch gekennzeichnet, dass** das generische Modell (1) durch eine Mittelwertbildung der Abtastwerte einer Vielzahl vermessener gleichartiger Strukturen ermittelt ist und die Komplexität eines Bereichs (A, B, C) des generischen Modells (1) basierend auf der Varianz der zu dem generischen Modell (1) gemittelten Abtastwerte der Strukturen in diesem Bereich ermittelt wird.

5. Verfahren nach einem der Ansprüche 1 bis 4, **dadurch gekennzeichnet, dass** das generische Modell (1) anhand von Vorregistrierungsdaten, die Landmarken (D, E, F G) der anatomischen Struktur des Patienten repräsentieren, an die anatomische Struktur des Patienten angepasst wird und die Komplexität eines Bereichs der anatomischen Struktur aus der Komplexität des korrespondierenden Bereichs des derart angepassten generischen Modells (1) ermittelt wird.

6. Verfahren nach einem der Ansprüche 1 bis 5, **dadurch gekennzeichnet, dass** die Konstellationsdaten eines Bereichs basierend auf einer Abbildung, beispielsweise einem Röntgenbild oder einer CT-Aufnahme, der anatomischen Struktur festgelegt werden.

7. Verfahren zum Morphen eines generischen Modells (1) einer anatomischen Struktur auf eine anatomische Struktur eines Patienten anhand von Messpunktdaten, die die anatomische Struktur an Messpunkten repräsentieren, **dadurch gekennzeichnet, dass** Konstellationsdaten, die die Konstellation der Messpunkte repräsentieren, anhand eines Verfahrens nach einem der Ansprüche 1 bis 6 bestimmt werden.

8. Verfahren nach Anspruch 7, **dadurch gekennzeichnet, dass** das generische Modell (1) an die Konstellationsdaten angepasst wird.

9. Softwareprogramm, das, wenn es auf einem Computer ausgeführt wird die Verfahrensschritte nach einem der Ansprüche 1 bis 8 ausführt.

10. Computerlesbares Medium, auf dem das Softwareprogramm nach Anspruch 9 gespeichert ist.

11. Vorrichtung (2) zum Festlegen von Konstellationsdaten, die die Anzahl und/oder den Abstand von Messpunkten auf einer anatomischen Struktur repräsentieren, aufweisend einen Computer (3), der dazu ausgebildet ist, das Verfahren nach einem der Ansprüche 1 bis 8 auszuführen.

## Claims

1. A method for determining arrangement data which represent the number of and/or distance between measurement points to be scanned on an anatomical structure of a patient, wherein the arrangement data are individually determined for different regions of the anatomical structure, wherein the method is a data processing method which is performed by a computer, and the arrangement data for a region of the anatomical structure are determined on the basis of the complexity of the respective region and/or at least one adjacent region, wherein the complexity is a value assigned to a region, and the higher the complexity of a region, the greater the number of measurement points and/or the smaller the distance between the measurement points which is selected in this region, **characterised in that** regions (A, B, C) of a generic grid network model (1) of the anatomical structure which correspond to the regions of the anatomical structure of the patient are determined, a region of the generic model is assigned a complexity, and the complexity of a region of the anatomical structure is equated with the complexity of the corresponding region of the generic model (1).

2. The method according to claim 1, **characterised in that** the generic model (1) consists of grid points, each grid point is assigned a complexity, and the complexity of a region (A, B, C) of the generic model (1) is ascertained from the complexity of the grid points in this region.

3. The method according to claim 1, **characterised in that** the generic model (1) consists of grid points, and the complexity of a region (A, B, C) of the generic model (1) is ascertained from the density of the grid points in this region.

4. The method according to any one of claims 1 to 3, **characterised in that** the generic model (1) is ascertained by averaging the scanning values of a multitude of homogenous surveyed structures, and the complexity of a region (A, B, C) of the generic model (1) is ascertained on the basis of the variance of the scanning values, averaged with respect to the generic model (1), of the structures in this region.

5. The method according to any one of claims 1 to 4, **characterised in that** the generic model (1) is adapted to the anatomical structure of the patient on the basis of pre-registration data which represent landmarks (D, E, F, G) of the anatomical structure of the patient, and the complexity of a region of the anatomical structure is ascertained from the complexity of the corresponding region of the generic model (1) adapted in this way.

6. The method according to any one of claims 1 to 5, **characterised in that** the arrangement data of a region are determined on the basis of an image, for example an x-ray image or a CT recording, of the anatomical structure.

7. A method for morphing a generic model (1) of an anatomical structure onto an anatomical structure of a patient on the basis of measurement point data which represent the anatomical structure at measurement points, **characterised in that** arrangement data which represent the arrangement of the measurement points are determined on the basis of a method according to any one of claims 1 to 6.

8. The method according to claim 7, **characterised in that** the generic model (1) is adapted to the arrangement data.

9. A software program which, when it is run on a computer, performs the method steps according to any one of claims 1 to 8.

10. A computer-readable medium on which the software program according to claim 9 is stored.

11. A device (2) for determining arrangement data which represent the number of and/or distance between measurement points on an anatomical structure, said device comprising a computer (3) which is designed to perform the method according to any one of claims 1 to 8.

## Revendications

1. Procédé pour déterminer des données en constellation qui représentent le nombre de points de mesure sur une structure anatomique d'un patient et/ou la distance entre les points de mesure à balayer, dans lequel les données en constellation sont déterminées individuellement pour différentes régions de la structure anatomique, dans lequel le procédé est un procédé de traitement de données qui est mis en oeuvre par un ordinateur et les données en constellation pour une région de la structure anatomique sont déterminées sur la base de la complexité de la région respective et/ou d'au moins une région adjacente, dans lequel la complexité est une valeur attribuée à une région et plus la complexité d'une région est élevée, plus le nombre des points de mesure est respectivement grand et plus la distance entre les points de mesure dans cette région est choisie petite, **caractérisé en ce que** des régions (A, B, C) d'un modèle de quadrillage générique (1) de la structure anatomique sont déterminées, lesquelles régions correspondent aux régions de la structure anatomique du patient, une complexité est attribuée à une région du modèle générique du patient, et la complexité d'une région de la structure anatomique est fixée égale à la complexité de la région correspondante du modèle générique (1).

2. Procédé selon la revendication 1, **caractérisé en ce que** le modèle générique (1) est constitué de points de grille, une complexité est attribuée à chaque point de grille et la complexité d'une région (A, B, C) du modèle générique (1) est déterminée à partir de la complexité des points de grille dans cette zone.

3. Procédé selon la revendication 1, **caractérisé en ce que** le modèle générique (1) est constitué de points de grille et la complexité d'une région (A, B, C) du modèle générique (1) est déterminée à partir de la densité des points de grille dans cette région.

4. Procédé selon l'une quelconque des revendications 1 à 3, **caractérisé en ce que** le modèle générique (1) est déterminé en mettant en moyenne les valeurs de balayage d'une pluralité de structures analogues examinées et la complexité d'une région (A, B, C) du modèle générique (1) est déterminée sur la base de la variance des valeurs de balayage de la structure dans cette région, lesquelles valeurs de balayage étant mises en moyenne par rapport au modèle générique (1).

5. Procédé selon l'une quelconque des revendications 1 à 4, **caractérisé en ce que** le modèle générique (1) est adapté à la structure anatomique du patient, sur la base de données de pré-enregistrement qui représentent des points de repère (D, E, F, G) de la structure anatomique du patient, et la complexité d'une région de la structure anatomique est déterminée à partir de la complexité de la région correspondante du modèle générique (1) adapté de cette manière.

6. Procédé selon l'une quelconque des revendications 1 à 5, **caractérisé en ce que** les données en constellation d'une région sont déterminées sur la base d'une image, par exemple d'une image de rayons X ou d'un enregistrement CT de la structure anatomique.

7. Procédé pour transposer par morphisme un modèle générique (1) d'une structure anatomique à une structure anatomique d'un patient sur la base de données de points de mesure qui représentent la structure anatomique sur des points de mesure, **caractérisé en ce que** des données en constellation qui représentent la constellation des points de mesure, sont déterminées sur la base d'un procédé selon l'une quelconque des revendications 1 à 6.

8. Procédé selon la revendication 7, **caractérisé en ce que** le modèle générique (1) est adapté aux données en constellation.

9. Programme logiciel qui, lorsqu'il est exécuté sur un ordinateur, exécute les étapes de procédé selon l'une quelconque des revendications 1 à 8.

10. Support lisible par ordinateur sur lequel est mémorisé le programme logiciel selon la revendication 9.

11. Dispositif (2) pour déterminer des données en constellation qui représentent le nombre de points de mesure sur une structure anatomique et/ou la distance entre des points de mesure, comportant un ordinateur (3) configuré pour mettre en oeuvre le procédé selon l'une quelconque des revendications 1 à 8.
